# EUROPEAN PATENT SPECIFICATION

(11) **EP 3 897 560 B1**
(45) Date of publication and mention of the grant of the patent: **29.06.2022**
(21) Application number: 18827021.9
(22) Date of filing: 17.12.2018
(51) Int. Cl.: A61K 8/42, A61Q 19/00, A61K 8/92, A61K 8/04

(54) **COMPOSITION COMPRISING OXALAMIDE GELATORS AND VEGETABLE OIL**
ZUSAMMENSETZUNG MIT OXALAMIDGELATOREN UND PFLANZENÖL
COMPOSITION COMPRENANT DES AGENTS DE GÉLIFICATION OXALAMIDE ET DE L'HUILE VÉGÉTALE

(43) Date of publication of application: 27.10.2021
(73) Proprietor: Rudjer Boskovic Institute, 10000 Zagreb (HR)
(72) Inventor: SIJAKOVIC VUJICIC, Natasa, 10000 Zagreb (HR); JERIC, Ivanka, 10000 Zagreb (HR); SUC SAJKO, Josipa, 10000 Zagreb (HR); RADOSEVIC, Petra, 10000 Zagreb (HR)
(74) Representative: Hadzija, Tomislav
(86) International application number: PCT/EP2018/085216
(87) International publication number: WO 2020/125926

(56) References cited:
- EP-A1- 1 013 264
- EP-A1- 2 365 052
- WO-A1-2005/047231
- WO-A1-2017/174615
- WO-A1-2017/194980
- WO-A2-2008/085360
- WO-A2-2009/030964
- JP-A- 2000 239 649

## Description

### FIELD OF THE INVENTION

The invention relates to the composition with gelling properties comprising low molecular weight gelators of Formula (I), vegetable oil and optionally a water. The invention further relates to the use of such composition in the food, cosmetic or pharmaceutical industry.

### BACKGROUND OF THE INVENTION

The food industry intensively searches for a new effective way of structuring edible oils, to prevent potential health issues. The World Health Organization (WHO) indicates the importance of controlling the intake of high levels of saturated fatty acids compared to reduced intake of mono- and poly-unsaturated fatty acids. However, saturated fatty acids are indispensable in food products, especially margarine, spreads, meat products and confectionery products, where they control key physical properties of the product (solid state, texture, spreadability and taste).

The conventional approach for structuring oil that is currently used in the food industries relies on the use of crystalline triacylglycerols (TAGs). These high melting TAGs are rich in saturated or even trans- fatty acids and the nutritional profile of this kind of structured oil systems is unhealthy. Also, structuring with this approach demands higher fractions of TAGs (till 20 wt %) compared with alternative methods. Alternative approaches that are based on the use of non-TAG structurants are nowadays explored to achieve efficient structuring of oil at comparatively lower concentrations.

It is necessary to distinguish different approaches of oil structuring through usage of low molecular weight organic compounds, polymeric compounds or inorganic compounds. Also, structuring of oil is possible through formation of network of crystalline particles, polymeric strands and self-assembled gel fibers.

Patent document WO 2005/047231 discloses trisubstituted cyclic thickening or gelator agents.

As reviewed in the papers of Patel and co-workers (summarized in book Ed. Richard W. Hartel, Alternative Routes to Oil Structuring, Springer, 2015 and the book Edible Oil Structuring, 2018, RSC, edited by A. R. Patel) many different approaches of oil structuring have been explored. Nowadays, from literature data it is obvious that different branches of industry are still looking for the best solution of structuring edible oils without addition of palm oil or different saturated fats to cause solidification of different products (Ashok R. Patel, Koen Dewettinck, Food Funct., 2016, 7, 20). Further, compounds capable of structuring oil phase at tremendously low concentration and in the same time emulsifying water in oil phase causing hardening (gelation) of different w/o emulsions with prolonged period of stability are still not known in the literature.

The initial studies dealt mostly with the use of lipidic additives (such as long chain fatty acids, fatty alcohols, dicarboxylic acids, wax esters, hydroxylated fatty acids, natural waxes and partial glycerides) as structuring agents of edible oil via direct dispersion at elevated temperatures followed by cooling. One example in this field concerns about lipid composition with structuring agent comprises at least 10 wt % of diacyclglycerols having a very long chain saturated fatty acid residue (WO2014/184118A1).

There are also few publications demonstrating two-component mixtures that showed synergistic gelling functionality such as stearic acid + stearyl alcohol, β -sitosterol + oryzanol, lecithin + sorbitan tristearate and mixtures of ceramides (E. D. Co and A. G. Marangoni, J. Am. Oil. Chem. Soc. 2012, 89, 749-780). Thereafter, a polymers such as modified cellulose (ethyl cellulose, EC) and proteins were also studied to extend the structuring principle beyond the crystalline network formation as seen with the lipid-based materials. In recent years, research on hydrophilic polymer-based gels, resin wax (shellac wax) gels and inorganic particle-based gels have also been published (J. Am. Oil. Chem. Soc. 2015, 92, 801-811; Eur. J. Lipid Sci. Technol., 2015, 117, 1772-1781). The natural waxes are approved as indirect additives, since there are regulatory concerns which need to be addressed. The major problem is tendency of waxes to post crystallization processes and instability of wax-based gels for longer storage period. Polymers such as cellulose derivatives, proteins and other hydrophilic polysaccharides are less feasible for scale-up, since there are additional processing steps required for gelation of oil (high temperature treatment, lyophilisation). There have been identified many potential structurants of oil, but there is still a need to find a food-grade oleogelator that is economical, efficient at low concentration, tolerant to processing conditions, compatible with the final composition of a product and primarily having a potent thixotropic property.

Structuring of oil through gelation phenomenon can serve as replacement of solid fats in both water-free (shortenings and chocolates) and water-containing (margarine, spreads and cooked meat products) products.

The main functional role of gelator agents in food formulation would be to provide structure and stabilization of the final product, possibility for controlled delivery of nutraceuticals and controlling of oil mobility and migration (allowing stability to chocolate products).

Organogels have many different functionalities in food products, including restriction of oil mobility and stabilization of water in oil (w/o) emulsions, especially in case were water droplets are entrapped inside the oleogel network.

There has been a great study on low-molecular-weight organic gelators (LMWOG) over the past decades, because of its academic interests and potential applications in cosmetics, foods, medical and pharmaceutical, photonic and electronic devices.

Through intensive research during the last two decades, more than 1000 structurally different low-molecular weight organic gelators (LMWOG) were shown to exhibit gelling ability toward various organic solvents and water. Although many low-molecular-weight gelators have been discovered and a few low-molecular-weight gelators have been used in cosmetics and commodities, their market shares are small, compared with polymer gelators.

In pharmaceutical industry, the LMWOG were explored mostly as hydrogelators for controlled release drug delivery. There are few examples of organogelators in pharmaceutical industry where LMWOG were explored for controlled release of bioactive substances (WO2009095485, US2005031650A1). Use of organogels in cosmetics is described in US2003091520.

Gel fibers, usually of micrometres scale lengths and nanometres scale diameters, are formed in solution through unidirectional self-assembly of gelator molecules. Such gels consist of a large amount of solvent and a very small amount of gelator molecules. The solvent is entrapped within the 3D network of entangled nanosize fibrous gelator aggregates. Due to the weak noncovalent interactions (hydrogen bonding, *π*-*π* stacking, van der Waals interactions and electrostatic interactions) that stabilize aggregates, most of the gels exhibit thermoreversible gel-to-sol transitions.

The precise relationship between the gelator structure (constitution, configuration and conformation), the properties of a solvent used to be gelled, and the motif of supramolecular organization within the gel fibers still remains to be discovered. Even very small variations of gelator constitution and changes of configuration can tremendously influence the gelation properties. Gelator stereochemistry has a noticeable influence on the gelation properties. For chiral gelators, both enantiomers have equal gelation properties, but symmetrical meso-diastereoisomers lack any gelation. In most cases, racemates are less efficient gelators than pure enantiomers, and sometimes lack any gelation ability.

A thixotropic supramolecular gel, which repeatedly undergoes the gel-to-sol transition by shearing and then sol-to-gel transition by standing, is a promising material. In spite of the many needs from industrial fields, it is very difficult to prepare such a gel. Although some thixotropic supramolecular gels have been reported, thixotropic supramolecular gels are serendipitously discovered with an amount less than a 1% in total.

Some of the compounds of the present invention have been described as organogelators for different organic solvents, commercial fuels and water (J. Makarević, M. Žinić, WO2009/030964). They have never been examined for gelation of vegetable oils or similar mixtures nor they showed thixotropic behaviour in examined organic solvents, commercial fuels and water. It has been now surprisingly found that the compounds of the present invention may be used as superorganogelators of different vegetable oils. Additionally, the organogelators of present invention show self-healing (thixotropic) properties in vegetable oils. Also, they can be classified as superorganogelators of vegetable oils since they have ability to form gels in vegetable oil till concentration 0.02 wt%. Further, the compounds show gelation of different mixtures of oil and water with similar viscoelastic properties to gels of pure vegetable oil. Tremendously, gels of water in oil emulsion showed also thixotropic properties. The gelation of oil at such low concentration of gelator molecules is very rare. The composition of present invention is the unique example of water/oil gelled emulsion systems with adorable viscoelastic properties applicable in wide range of industries.

Compounds disclosed in WO2017194980 have been described as gelators of vegetable oils. The compounds of the present invention represent further solution for gelation of vegetable oils with additional advantages such as capability to form water in oil gelled emulsion, better thixotropic properties and more powerful efficiencies.

The powerful applicability of vegetable oil gelators and especially w/o emulsion gelators is envisaged in food (specifically in oil and meat industry), cosmetic and pharmaceutical industry.

### SUMMARY OF THE INVENTION

The present invention relates to the composition with gelling properties comprising a compound of Formula (I) 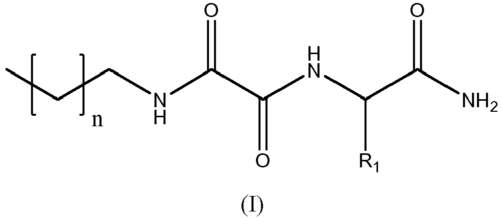 and a vegetable oil;
wherein R¹ is selected from -CH₃, -CH(CH₃)₂, -CH₂CH(CH₃)₂, -CH(CH₃)CH₂CH₃, -CH₂Ph and -Ph;
n is an integer from 0-14.

The present invention further relates to the use of the composition comprising a compound of formula (I) and the oil, in food, cosmetic or pharmaceutical industry.

### BRIEF DESCRIPTION OF THE FIGURES

**Figure 1****.** Thixotropic behaviour of the sunflower gel based on gelator 6-L at 0,1 wt%;(a) Gel formed from hot solution upon cooling; (b, c) Low-viscosity fluid formed by vigorous hand shaking; (d) Gel reformed after standing for 5 min at room temperature.
**Figure 2****.** Thixotropic behaviour of the 0.1 wt% **6-L** gelled w/o emulsion of the sunflower oil with 10 % of water; (a) Gel formed from hot solution upon cooling; (b, c) The gel reformed after a vigorous hand shaking at room temperature (2 consecutive cycles).
**Figure 3.** a) The **6-L** gelled olive oil; b) the **6-L** gelled w/o emulsion with 50 % of water in sunflower oil.
**Figure 4****.** Amplitude sweeps (G' and G" values) of 0.2 wt % of **4-L**, **6-L**, **8-L** and **12-L** sunflower oil gels at 15 °C.
**Figure 5****.** Amplitude sweeps (G' and G" values) of 0.2 wt % of **4-L**, **6-L**, **8-L** and **12-L** gelators in a gelled water in sunflower oil emulsion (10% water) at 15 °C.
**Figure 6****.** Amplitude sweeps (G' and G" values) of 0.2 wt % of **4-A**, **4-L**, **4-V** and **4-P** sunflower oil gels at 15 °C.
**Figure 7****.** Frequency sweeps (G' (■) and G" (▲) values) for 0.2 wt % of **4-L**; a) the sunflower oil gel (solid line) and b) the gelled water in oil emulsion (10 % water; dotted line) at 15 °C.
**Figure 8****.** Recovery of G' (■) and G" (A) values (2 consecutive thixotropic recovery cycles) at 15 °C for 0.2 wt % of the **6-L** sunflower oil gel (grey) and gelled w/o emulsion (10 % of water) (black) as a function of time and application of different strains. Linear viscoelastic region: γ = 0.1%, ω = 10 rad/s. Destructive strain: γ=100%, ω=10 rad/s.

### DETAILED DESCRIPTION OF THE INVENTION

The following abbreviations are used in the text: TEA for thriethylamine, Boc for tert-butyloxycarbonyl protecting group; iBu for isobutyl; Et for ethyl; DCM for dichlormetan; NMM for N-methylmorpholine; TFA for trifluoroacetic acid; Ph for phenyl.

Aminoacids: Ala for Alanine, Val for Valine, Leu for Leucine, ILe for Isoleucine, Phe for Phenylalanine, Phg for Phenylglycine.

Organogelators or gelling agents represent molecules capable to self-assemble in different solvents into three-dimensional nano-network of self-assembled fibers through highly specific noncovalent interactions such as hydrogen bonding, van der Waals, p-stacking, electrostatic and charge-transfer interactions.

Organogels represent mixture of gel fibers present in solid state and liquid phase entrapped in pores between self-assembled fibers.

"Organogel of the present invention" or "organogel of the invention" as used herein refers to a composition comprising a compound of formula (I) and oil as liquid phase structured into gel state. Vegetable oils encompassed by present invention are edible oils or base cosmetic oils.

Edible oils are selected from palm oil, sunflower oil, olive oil, soybean oil, linseed oil, rapeseed oil, corn oil, pumpkin seed oil, sesame oil, safflower oil, castor, peanut oil and the like.

The cosmetic base oils are selected from sweet almond oil, linseed oil, grape seed oil, avocado oil, apricot oil, olive oil, sesame oil, rapeseed oil, sunflower oil, jojoba oil, castor oil, borage seed oil, argan oil, calendula oil, evening primrose oil, hazelnut oil, walnut oil, peanut oil, macadamia oil, coconut oil, rose hip seed oil, wheat germ oil, St. John's wort oil, blueberry seed oil, black cumin seed, rice bran oil and the like.

"A composition according to present invention" or "a composition according to the invention" as used herein relates to the composition comprising the compound of formula (I) and a vegetable oil.

"A compound of the present invention" or "a compound of the invention" as used herein relates to the compound of formula (I).

The term "thixotropy" as used herein refers to the property of certain gels that are thick (viscous) under normal conditions, but flow (become thin, less viscous) over time when shaken, agitated, or otherwise stressed and then take a fixed time to return to a gel state. Low molecular weight organic gelators in general are extremely sensitive to mechanical stress and these systems irreversibly expel solvent from their gel network when subjected to flow. On removal of the external force, these systems lose its original elastic properties. On the other hand, thixotropic gels can disintegrate in solution under an external mechanical stress and can regain their elastic properties upon removal of the stress. This operation can be carried out for an infinite number of cycles. Thixotropic LMOGs represent a unique class of dynamic self-assembled supramolecular systems.

An emulsion is termed an oil/water (o/w) emulsion if the dispersed phase is a liquid (an oil) and the continuous phase is water or an aqueous solution and is termed water/oil (w/o) if the dispersed phase is water or an aqueous solution and the continuous phase is an organic liquid (an oil).

The term "0.2 wt % 6-L sunflower oil gel" or a similar expressions mean for a sunflower oil gel containing 0.2 wt % of the compound 6-L.

In one aspect, compounds of the present invention are organogelators of vegetable oil.

In one aspect the present invention relates to the use of a compound of the present invention as an organogelator of vegetable oil.

In one aspect the present invention relates to a composition comprising a compound of formula (I) and a vegetable oil.

In one aspect of the present invention composition is in the form of a gel.

In one aspect the present invention relates to a composition comprising a compound of formula (I), a vegetable oil and a water.

In one aspect of the present invention composition is gelled water in oil emulsion.

With regard to stereoisomers, the compounds of Formula (I) have one asymmetric carbon atom. Thus, the compounds of Formula (I) may occur as individual enantiomers, or mixtures thereof. All enantiomers are included within the present invention.

In the following description, R¹ and n have the meaning as defined for the compounds of Formula (I) unless otherwise stated.

It will be understood that the present invention covers all combinations of aspects, suitable, convenient and preferred groups described herein.

In one aspect the present invention relates to the composition comprising a compound of formula (Ia)

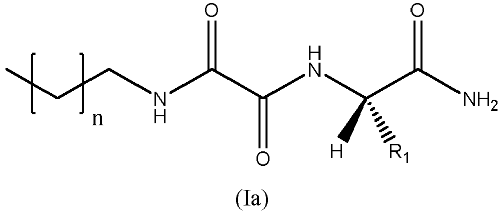

In one aspect the present invention relates to the composition comprising a compound of formula (I) or (Ia) and a vegetable oil wherein n is an integer from 2-10.

In further aspect the present invention relates to the composition comprising a compound of formula (I) or (Ia) and a vegetable oil wherein n is 2, 4, 6 or 10.

In one aspect the present invention relates to the composition comprising a compound of formula (I) or (Ia) and a vegetable oil wherein R¹ is selected from -CH₃, -CH(CH₃)₂, -CH₂CH(CH₃)₂, - CH(CH₃)CH₂CH₃ and -CH₂Ph.

In one aspect the present invention relates to the composition comprising a compound of the invention and a vegetable oil.

In further aspect the vegetable oil is edible oil.

In one further aspect the edible oil is selected from palm oil, sunflower oil, olive oil, soybean oil, linseed oil, rapeseed oil, corn oil, pumpkin seed oil, sesame oil, safflower oil, castor, peanut oil and combination thereof. In yet further aspect the edible oil is selected from sunflower oil, olive oil, soybean oil, rapeseed oil, palm oil, and combination thereof. In yet further aspect the edible oil is selected from sunflower oil, olive oil, soybean oil and combination thereof.

In one further aspect the present invention relates to the composition comprising a compound of the invention and a cosmetic base oil. In further aspect the cosmetic base oil is selected from sweet almond oil, linseed oil, grape seed oil, avocado oil, apricot oil, olive oil, sesame oil, rapeseed oil, sunflower oil, jojoba oil, castor oil, borage seed oil, argan oil, avocado oil, calendula oil, evening primrose oil, hazelnut oil, walnut oil, peanut oil, macadamia oil, coconut oil, rose hip seed oil, wheat germ oil, St. John's wort oil, blueberry seed oil, black cumin seed, rice bran oil or combination thereof. In yet further aspect the cosmetic base oil is selected from almond oil, avocado oil, jojoba oil, coconut oil, rice bran oil, peanut oil.

In one aspect the present invention relates to the composition comprising a compound of the invention and a vegetable oil wherein the compound of the invention is present in the composition in the concentration at which fluid motion of the oil stops.

In one aspect the present invention relates to the composition comprising a compound of the invention and a vegetable oil wherein the compound of the invention is present in the composition in the concentration at which gel is forming.

In one aspect the compound of the invention is present in the composition at a concentration of about 20% or less, on a weight/weight basis. In another aspect, the compound of the invention is present in the composition at a concentration of about 10%, on a weight/weight basis. In yet another aspect, the compound of the invention is present in the composition at a concentration of about 5% or less, on a weight/weight basis. In another aspect, compound of the invention is present in the composition at a concentration of about 2%, on a weight/weight basis. In yet another aspect, the compound of the invention is present in the composition at a concentration of about 2% or less, on a weight/weight basis. In yet another aspect, the compound of the invention is present in the composition at a concentration of about 0.5% or less, on a weight/weight basis. In yet another aspect, the compound of the invention is present in the composition at a concentration of about 0.05% or less, on a weight/weight basis.

In one aspect, the compound of the invention is present in the composition in the concentration of about 0,02 to about 10 wt % relative to the total weight of the composition.

In one aspect the present invention relates to the composition comprising a compound of the invention, a vegetable oil and water.

In one aspect water is present in the composition in the concentration to about 10 wt % relative to the total weight of the composition. In further aspect water is present in the composition in the concentration to about 20 wt % relative to the total weight of the composition. In further aspect water is present in the composition in the concentration to about 30 wt % relative to the total weight of the composition. In further aspect water is present in the composition in the concentration to about 40 wt % relative to the total weight of the composition. In further aspect water is present in the composition in the concentration to about 50 wt % relative to the total weight of the composition. In further aspect water is present in the composition in the concentration to about 80 wt % relative to the total weight of the composition.

In one aspect, the composition according to present invention forms an organogel.

In one aspect the composition according to present invention exhibits a thixotropic property.

In another aspect, the organogel recovers at least about 70% within less than about 10 minutes, preferably within less than about 5 minutes, and more preferably within less than about 1 minute after the exposure to destructive shear. In still another embodiment, the organogel recovers at least about 80% within less than about 10 minutes, preferably within less than about 5 minutes, and more preferably within less than about 1 minute after the exposure to destructive shear. In a further aspect, the organogel recovers at least about 90% within less than about 10 minutes, preferably within less than about 5 minutes, and more preferably within less than about 1 minute after the exposure to destructive shear.

In another aspect, a gelled w/o emulsion recovers at least about 70% within less than about 10 minutes, preferably within less than about 5 minutes, and more preferably within less than about 1 minute after the exposure to destructive shear. In still another embodiment, the organogel recovers at least about 80% within less than about 10 minutes, preferably within less than about 5 minutes, and more preferably within less than about 1 minute after the exposure to destructive shear. In a further aspect, the organogel recovers at least about 90% within less than about 10 minutes, preferably within less than about 5 minutes, and more preferably within less than about 1 minute after the exposure to destructive shear.

In one aspect, the composition according to present invention may be used in the pharmaceutical industry as vector/carriers for active substances.

In one aspect the present invention relates to the composition comprising a compound of the present invention, edible oil and the active pharmaceutical substance. In further aspect active pharmaceutical substance are provided for a sustained release.

In one aspect the present invention relates to the composition comprising a compound of the invention, edible oil and the nutraceuticals substance wherein the nutraceuticals substance refers to dietary supplements such as vitamins, minerals, fatty acids, amino acids, proteins, herbal medicine etc.

In one aspect the invention relates to a composition comprising a compound of the invention, edible oil and a food. In further aspect food is selected from shortenings and chocolates. In yet further aspect food is selected from margarine, spreads and cooked meat products.

In another aspect, the invention relates to a cosmetic composition comprising at least one cosmetically acceptable ingredient, a compound of the invention and base cosmetic oil.

In one aspect, the invention relates to a composition usable as a cleaning tool in cultural heritage conservation comprising a compound of the invention and oil.

In one aspect, the invention relates to a consumer product comprising a compound of the invention and a vegetable oil. In further aspect consumer product is lubricant.

In one aspect the invention covers process for preparing the composition of the present invention comprising the following steps:
(a) mixing the compounds of formula (I) and the vegetable oil;
(b) heating the mixture obtained in step (a) to a temperature until compound is completely dissolved;
(c) cooling the mixture obtained in step (b) to room temperature or bellow.

In one aspect the invention covers process for preparing the composition of the present invention comprising the following steps:
(a) mixing the compounds of formula (I) and the vegetable oil;
(b) heating the mixture obtained in step (a) to a temperature until compound is completely dissolved;
c) adding and mixing a specific amount of water;
(d) cooling the mixture obtained in step (c) to room temperature or bellow.

In one aspect the invention covers process for preparing the composition of the present invention comprising the following steps:
(a) mixing the food, pharmaceutically active ingredient, nutraceuticals or a cosmetic ingredient as solid or solubilised component in oil or water;
(b) heating the mixture obtained in step (a) to a temperature until ingredient is completely dissolved;
(c) adding and mixing the compounds of formula (I) dissolved in oil with mixtures obtained during step (a) or step (b)
(d) cooling the mixture obtained in step (b) to room temperature or bellow.

In one aspect the invention covers process for preparing the composition of the present invention comprising the following steps:
(a) mixing the compounds of formula (I) and the vegetable oil;
(b) heating the mixture obtained in step (a) to a temperature until compound is completely dissolved;
optionally (c) mixing the food, pharmaceutically active ingredient, nutraceuticals or a cosmetic ingredient as solid or solubilised component in oil or water into the mixture during the step (b); (d) cooling the mixture obtained in step (b) to room temperature or bellow.

Further alternatively, a food, a pharmaceutically active ingredient, a nutraceuticals or a cosmetic ingredient as solid or solubilised component in oil or water may be added to the mixture after or during step (d).

In one aspect the invention covers process for preparing the composition of the present invention comprising the following steps:
(a) mixing the compounds of formula (I) and the vegetable oil; and optionally a food, pharmaceutically active ingredient, nutraceuticals or a cosmetic ingredient;
(b) heating the mixture obtained in step (a) to a temperature until compound is completely dissolved;
(c) heating of water soluble ingredients in a water phase;
(d) mixing of heated oil phase obtained in (b) and water phase obtained in (c);
(e) cooling the mixture obtained in step (d) to room temperature or bellow.

### Method of preparation:

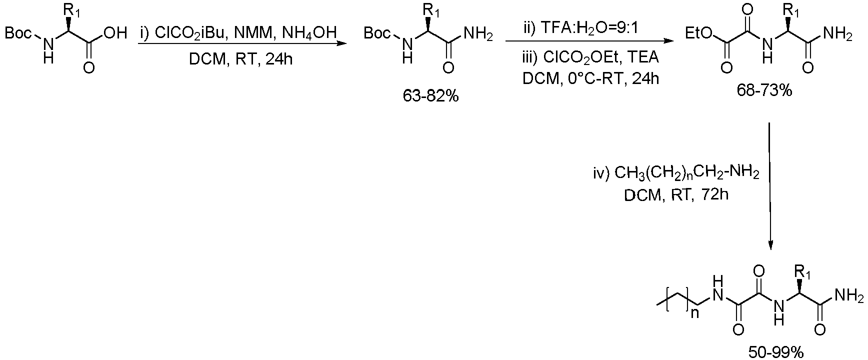

Compounds of Formula (I) may be prepared by the general methods outlined hereinafter.

Compounds of Formula (I) may be prepared from ester of formula (II) 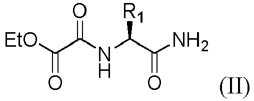 by reaction with amine of formula (III) in the presence of DCM at room temperature for 72 h.

CH₃(CH₂)ₙCH₂-NH₂ (III)

Oxalamide ester of Formula (II) may be prepared starting from protected amide of formula (IV) 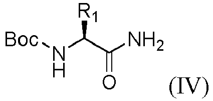 in the presence of mixture TFA:H₂O (9:1) and finally by reaction with ethyl oxalyl chloride of formula ClCO₂OEt and TEA in DCM as solvent at 0 °C for 24 hours.

Amide of formula (IV) may be prepared starting from protected amino acid of formula (V) 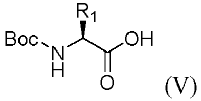 by amide formation in the presence of ClCO₂iBu, NMM and ammonium hydroxide in DCM as solvent.

Protected amino acids of formula (V) are commercially available.

### Examples

Preparation of Intermediates

| 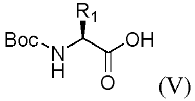 | R¹ |
|---|---|
| Boc-Ala-OH | -CH₃ |
| Boc-Val-OH | -CH(CH₃)₂ |
| Boc-Leu-OH | -CH₂CH(CH₃)₂ |
| Boc-Ile-OH | -CH(CH₃)CH₂CH₃ |
| Boc-Phe-OH | -CH₂Ph |

### Synthesis of protected amide of formula BocNHCH(R¹)C(=O)NH₂

To a BocNHCH(R¹)C(=O)OH (1 mmol) dissolved in dry DCM (10 mL) was added NMM (1 mmol) and solution cooled down to 0 °C. Isobutyl chloroformate (1 mmol) was added dropwise and reaction was stirred for additional 15 min at 0 °C. Ammonia solution (5 mL) was added and reaction was stirred at room temperature overnight. The consumption of starting amino acid was monitored by TLC. The solvent was evaporated and white precipitate was dissolved with EtOAc and washed with brine and water. Organic layer was evaporated and product purified by recrystallisation using EtOAc/hexane. The Intermediate products (IV) were obtained as a white solid (63-82% yield).

| 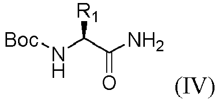 | R¹ | yield |
|---|---|---|
| Intermediate **Boc-Ala-NH₂** | -CH₃ | 2.51 g; 63 % |
| Intermediate **Boc-Val-NH₂** | -CH(CH₃)₂ | 3.00 g; 77 % |
| Intermediate **Boc-Leu-NH₂** | -CH₂CH(CH₃)₂ | 3.60 g; 65 % |
| Intermediate **Boc-Ile-NH₂** | -CH(CH₃)CH₂CH₃ | 4.10 g; 82 % |
| Intermediate **Boc-Phe-NH₂** | -CH₂Ph | 3.10 g; 77 % |

### Synthesis of TFA salt of formula TFA⁻⁺H₃NCH(R¹)C(=O)NH₂

BocNHCH(R¹)C(=O)NH₂ (Intermediate (IV)) was dissolved in TFA-H₂O mixture (9:1, v/v) and the reaction was stirred at room temperature for 1h. The consumption of starting material was monitored by TLC. Reaction mixture was added dropwise to a cold diisopropyl ether which precipitate final product in quantitative yield as a white crystalline solid used in the next step.

### Synthesis of ester of formula EtOC(=O)C(=O)NHCH(R¹)C(=O)NH₂

TFA⁻⁺H₃NCH(R¹)C(=O)NH₂ (1 mmol) was dissolved in dry DCM (10 mL) with the addition of TEA (2 mmol). Reaction mixture was cooled down to 0 °C and ClCO₂OEt (1 mmol) was added dropwise through 15 min and reaction was stirred for additional 30 min at 0 °C. Reaction was warmed at room temperature and stirred overnight. The consumption of starting material was monitored by TLC. The solvent was evaporated and white precipitate was dissolved with EtOAc and washed with 5% HCl, 10% NaHCO₃, brine and water. Organic layer was evaporated and product purified by recrystallisation using EtOAc/hexane. The intermediate product (II) was obtained as a white solid (27-73% yield).

| 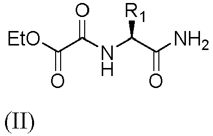 | R¹ | yield | ¹H NMR (600 MHz, DMSO) δ / ppm | ¹³C NMR (151 MHz, DMSO) δ / ppm |
|---|---|---|---|---|
| **Intermediate EtO-oxalyl-Ala-NH₂** | -CH₃ | 0.67 g; 27% | 8.67 (d, *J* = 7.4 Hz, 1H), 7.49 (s, 1H), 7.10 (s, 1H), 4.26- 4.19 (m, 3H), 1.30- 1.25 (m, 6H) | 173.5, 160.9, 157.0, 62.5, 48.9, 18.3, 14.3 |
| **Intermediate EtO-oxalyl-Val-NH₂** | -CH(CH₃)₂ | 2.20 g; 68% | 8.35 (d, *J* = 8.9 Hz, 1H), 7.54 (s, 1H), 7.18 (s, 1H), 4.24 (q, *J* = 7.1 Hz, 2H), 4.12 (dd, *J* = 8.9, 7.0 Hz, 1H), 2.08-1.99 (m, 1H), 1.33 - 1.24 (m, 3H), 0.88 (d, J = 6.8 Hz, 3H), 0.84 (d, *J* = 6.8 Hz, 3H) | 172.2, 161.3, 157.7, 62.5, 58.5, 30.9, 19.6, 18.4, 14.3 |
| **Intermediate EtO-oxalyl-Leu-NH₂** | -CH₂CH(CH₃)₂ | 2.06 g; 70% | 8.69 (d, *J* = 8.5 Hz, 1H), 7.45 (s, 1H), 7.06 (s, 1H), 4.27 - 4.22 (m, 3H), 1.66- 1.59 (m, 1H), 1.58- 1.46 (m, 2H), 1.27 (t, *J* = 7.1 Hz, 3H), 0.87 (d, *J* = 6.3 Hz, 3H), 0.84 (d, *J* = 6.2 Hz, 3H) | 173.4, 161.0, 157.5, 62.4, 51.9, 40.8, 24.8, 23.4, 21.6, 14.3 |
| **Intermediate** | -CH(CH₃)CH₂CH₃ | 2.00 g; 71 % | 8.38 (d, *J=* 8.9 Hz, 1H), 7.54 (s, 1H), 7.16 | 172.3, 161.3, |
| **EtO-oxalyl-ILe-NH₂** | | | (s, 1H), 4.23 (q, *J* = 7.1 Hz, 2H), 4.13 (t, *J* = 8.2 Hz, 1H), 1.80 (d, *J* = 5.9 Hz, 1H), 1.43 (m, 1H), 1.27 (t, *J* = 7.1 Hz, 3H), 1.11 - 1.02 (m, 1H), 0.87 - 0.80 (m, 6H) | 157.6, 62.5, 57.7, 36.9, 24.8, 15.8, 14.3, 11.3 |
| **Intermediate EtO-oxalyl-Phe-NH₂** | -CH₂Ph | 2.20 g; 73 % | δ 8.75 / ppm (d, *J* = 8.5 Hz, 1H), 7.59 (s, 1H), 7.28 - 7.21 (m, 6H), 4.48 - 4.42 (m, 1H), 4.20 (q, *J* = 7.0 Hz, 2H), 3.13 - 3.10 (m, 1H), 3.01 - 2.97 (m, 1H), 1.34 - 1.18 (m, 3H) | 172.3, 160.8, 157.2, 138.2, 129.6, 128.6, 126.8, 62.4, 54.8, 37.2, 14.2. |

### Synthesis of compounds of Formula (I)

To an EtOC(=O)C(=O)NHCH(R¹)C(=O)NH₂ (1 mmol) dissolved in dry DCM (10 mL) was added dropwise solution of CH₃(CH₂)ₙCH₂-NH₂ (1.7 mmol) in dry DCM (5 mL) and the reaction mixture was stirred at room temperature for 72 h. The consumption of starting material was monitored by TLC. The solvent was evaporated and white precipitate was dissolved with EtOAc and washed with 5% HCl, 10% NaHCO₃, brine and water. Organic layer was evaporated and product purified by recrystallisation using EtOAc/hexane. The final product of Formula (I) was obtained as a white solid (18-99% yield).

| 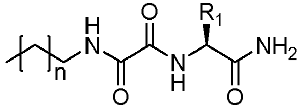 | R¹ | n | yield |
|---|---|---|---|
| **C₄-oxalyl-Ala-NH₂ (4-A)** | -CH₃ | 2 | 0.11 g; 18 % |
| **C₄-oxalyl-Val-NH₂ (4-V)** | -CH(CH₃)₂ | 2 | 0.37 g; 65 % |
| **C₅-oxalyl-Val-NH₂ (5-V)** | **-**CH(CH₃)₂ | 3 | 0.32 g; 54 % |
| **C₆-oxalyl-Val-NH₂ (6-V)** | -CH(CH₃)₂ | 4 | 0.50 g; 80 % |
| **C₈-oxalyl-Val-NH₂ (8-V)** | -CH(CH₃)₂ | 6 | 0.35 g; 50 % |
| **C₁₂-oxalyl-Val-NH₂ (12-V)** | -CH(CH₃)₂ | 10 | 0.44 g; 53 % |
| **C₄-oxalyl-Leu-NH₂ (4-L)** | -CH₂CH(CH₃)₂ | 2 | 0.51 g; 91% |
| **C₆-oxalyl-Leu-NH₂ (6-L)** | -CH₂CH(CH₃)₂ | 4 | 0.50 g; 81 % |
| **C₈-oxalyl-Leu-NH₂ (8-L)** | **-**CH₂CH(CH₃)₂ | 6 | 0.62 g; 92 % |
| **C₁₂-oxalyl-Leu-NH₂ (12-L)** | -CH₂CH(CH₃)₂ | 10 | 0.80 g; 90 % |
| **C₄-oxalyl-Ile-NH₂ (4-IL)** | -CH(CH₃)CH₂CH₃ | 2 | 0.50 g; 90 % |
| **C₆-oxalyl-Ile-NH₂ (6-IL)** | -CH(CH₃)CH₂CH₃ | 4 | 0.61 g; 99 % |
| **C₈-oxalyl-Ile-NH₂ (8-IL)** | -CH(CH₃)CH₂CH₃ | 6 | 0.63 g; 93 % |
| **C₁₂-oxalyl-Ile-NH₂ (12-IL)** | -CH(CH₃)CH₂CH₃ | 10 | 0.8 g; 99 % |
| **C₄-oxalyl-Phe-NH₂ (4-P)** | -CH₂Ph | 2 | 0.50 g; 91 % |
| **C₆-oxalyl-Phe-NH₂ (6-P)** | -CH₂Ph | 4 | 0.52 g; 87 % |
| **C₈-oxalyl-Phe-NH₂ (8-P)** | -CH₂Ph | 6 | 0.63 g; 96 % |
| **C₁₂-oxalyl-Phe-NH₂ (10-P)** | -CH₂Ph | 10 | 0.72 g; 94 % |

**C₄-oxalyl-Ala-NH₂ (4-A):** ¹HNMR (600 MHz, DMSO) δ /ppm 8.75 (s, 1H), 8.37 (d, J = 7.3 Hz, 1H), 7.48 (s, 1H), 7.14 (s, 1H), 4.28-4.18 (m, 1H), 3.18 - 3.08 (m, 2H), 1.47- 1.40 (m, 2H), 1.31 -1.22 (m, 5H), 0.87 (t, J = 7.3 Hz, 3H); ¹³C NMR (151 MHz, DMSO) δ / ppm 173.7, 160.1, 159.7, 48.7, 39.0, 31.2, 19.9, 18.8, 14.0; HRMS (MALDI TOF/TOF): calcd for C₉H₁₇N₃O₃ [M+H]⁺ 216,1348; found 216,1351.

**C₄-oxalyl-Val-NH₂** (**4-V):**¹H NMR (600 MHz, DMSO) δ / ppm 8.82 (t, *J=* 5.5 Hz, 1H), 8.08 (d, *J* = 9.3 Hz, 1H), 7.58 (s, 1H), 7.21 (s, 1H), 4.15 - 4.09 (m, 1H), 3.20-3.08 (m, 2H), 2.08- 1.98 (m, 1H), 1.50 - 1.40 (m, 2H), 1.31 - 1.20 (m, 2H), 0.87 (t, *J* = 7.3 Hz, 6H), 0.82 (d, *J* = 6.7 Hz, 3H); ¹³C NMR (151 MHz, DMSO) δ / ppm 172.3, 160.0, 159.9,58.3,39.1,31.4,31.2, 19.9, 19.7, 18.2, 14.0; HRMS (MALDI TOF/TOF): calcd for C₁₁H₂₁N₃O₃ [M+H]⁺ 244,1661; found 244,1669.

**C₅-oxalyl-Val-NH₂ (5-V):**¹H NMR (300 MHz, DMSO) δ / ppm 8.86 (t, *J* = 5.8 Hz, 1H), 8.10 (d, *J* = 9.4 Hz, 1H), 7.63 (s, 1H), 7.25 (s, 1H), 4.15 - 4.09 (m, 1H), 3.21 - 3.05 (m, 2H), 2.12 - 1.95 (m, 1H), 1.53 -1.41 (m,2H), 1.33 -1.18 (m, 4H), 0.90 - 0.80 (m, 9H); ¹³C NMR (75 MHz, DMSO) δ / ppm 172.3, 159.9, 159.9, 58.2, 31.4, 28.9, 28.8, 22.2, 19.7, 18.2, 14.3; HRMS (MALDI TOF/TOF): calcd for C₁₂H₂₃N₃O₃ [M+H]⁺ 258,1818; found 258,1821.

**C₆-oxalyl-Val-NH₂ (6-V):** ¹H NMR (600 MHz, DMSO) δ /ppm 8.82 (t, *J* = 5.7 Hz, 1H), 8.08 (d, *J* = 9.3 Hz, 1H), 7.59 (s, 1H), 7.21 (s, 1H), 4.15 - 4.09 (m, 1H), 3.18-3.07 (m, 2H), 2.12 - 1.95 (m, 1H), 1.50 - 1.41 (m, 2H), 1.30 - 1.20 (m, 6H), 0.90 - 0.86 (m, 6H), 0.82 (d, *J* = 6.7 Hz, 3H); ¹³C NMR (151 MHz, DMSO) δ / ppm 172.3, 160.0, 58.3, 31.3, 29.0, 26.4, 22.4, 19.7, 18.2, 14.3; HRMS (MALDITOF/TOF): calcd for C₁₃H₂₅N₃O₃ [M+Na]⁺ 294,1794; found 294,1790.

**C₈-oxalyl-Val-NH₂ (8-V):** ¹H NMR (600 MHz, DMSO) δ /ppm 8.80 (t, *J* = 5.6 Hz, 1H), 8.06 (d, *J* = 9.3 Hz, 1H), 7.56 (s, 1H), 7.19 (s, 1H), 4.15 - 4.09 (m, 1H), 3.15 - 3.06 (m, 2H), 2.05 - 1.97 (m, 1H), 1.47 - 1.40 (m, 2H), 1.30 - 1.20 (m, 10H), 0.89 - 0.83 (m, 6H), 0.80 (d, *J* = 6.7 Hz, 3H); ¹³C NMR (151 MHz, DMSO) δ 172.3, 160.0, 159.9, 58.3, 31.6, 31.4,29.1, 29.0, 26.7, 22.5, 19.7, 18.2, 14.4; HRMS (MALDI TOF/TOF): calcd for C₁₅H₂₉N₃O₃ [M+Na]⁺ 322,2107; found 322,2113.

**C₁₂-oxalyl-Val-NH₂ (12-V):** ¹H NMR (600 MHz, DMSO) δ 8.81 (t, *J=* 5.8 Hz, 1H), 8.08 (d, *J* = 9.3 Hz, 1H), 7.59 (s, 1H), 7.21 (s, 1H), 4.15 - 4.09 (m, 1H), 3.17 - 3.07 (m, 2H), 2.07 - 2.00 (m, 1H), 1.48 - 1.41 (m, 2H), 1.23 (s, 19H), 0.89 - 0.80 (m, 10H); ¹³C NMR (151 MHz, DMSO) δ 172.3, 160.0, 159.9, 58.2, 31.7, 31.4,29.5,29.4, 29.3,29.2,29.1, 29.0,28.9,26.7, 22.5, 19.7, 18.2, 14.4; HRMS (MALDI TOF/TOF): calcd for C₁₉H₃₇N₃O₃ [M+Na]⁺ 378,2733; found 378,2730.

**C₄-oxalyl-Leu-NH₂ (4-L):** ¹H NMR (600 MHz, DMSO) δ /ppm 8.76 (t, *J* = 5.9 Hz, 1H), 8.34 (d, *J* = 9.0 Hz, 1H), 7.47 (s, 1H), 7.08 (s, 1H), 4.30 - 4.24 (m, 1H), 3.18-3.08 (m, 2H), 1.64 - 1.59 (m, 1H), 1.54 - 1.48 (m, 2H), 1.48 - 1.40 (m, 2H), 1.29 - 1.22 (m, 2H), 0.89 - 0.84 (m, 9H); ¹³C NMR (151 MHz, DMSO) δ / ppm 173.6, 160.0, 51.8, 41.4, 39.1, 31.2, 24.8, 23.4, 21.9, 19.9, 14.0; HRMS (MALDI TOF/TOF): calcd for C₁₂H₂₃N₃O₃ [M+Na]⁺ 280,1637; found 280,1640.

**C₆-oxalyl-Leu-NH₂ (6-L);** ¹H NMR (600 MHz, DMSO) δ /ppm 8.76 (t, *J* = 5.8 Hz, 1H), 8.34 (d, *J* = 9.0 Hz, 1H), 7.46 (s, 1H), 7.08 (s, 1H), 4.30-4.23 (m, 1H), 3.15 - 3.07 (m, 2H), 1.62 (t, *J* = 9.4 Hz, 1H), 1.55 - 1.48 (m, 2H), 1.48 - 1.42 (m, 2H), 1.29 - 1.22 (m, 6H), 0.89 - 0.84 (m, 9H); ¹³C NMR (151 MHz, DMSO) δ / ppm 173.6, 160.1, 160.0, 51.8, 41.4, 39.4, 31.4, 29.1, 26.4, 24.8, 23.4, 22.4, 21.9, 14.3; HRMS (MALDI TOF/TOF): calcd for C₁₄H₂₇N₃O₃ [M+Na]⁺ 308,1950; found 308,1952.

**C₈-oxalyl-Leu-NH₂ (8-L):** ¹H NMR (300 MHz, DMSO) δ /ppm 8.76 (t, *J* = 5.8 Hz, 1H), 8.34 (d, *J* = 9.0 Hz, 1H), 7.46 (s, 1H), 7.09 (s, 1H), 4.31-4.18 (m, 1H), 3.15 - 3.07 (m, 2H), 1.65 - 1.55 (m, 1H), 1.48 - 1.42 (m, 4H), 1.22 (s, 10H), 0.89 - 0.84 (m, 9H); ¹³C NMR (75 MHz, DMSO) δ / ppm 173.6, 160.0, 159.9, 51.8,41.4, 31.7,29.1,26.8, 24.8,23.5,22.5, 21.9, 14.4; HRMS (MALDI TOF/TOF): calcd for C₁₆H₃₁N₃O₃ [M+Na]⁺ 336,2263; found 336,2256.

**C₁₂-oxalyl-Leu-NH₂ (12-L):** ¹H NMR (300 MHz, DMSO) δ / ppm 8.76 (t, *J=* 5.9 Hz, 1H), 8.34 (d, *J* = 9.0 Hz, 1H), 7.47 (s, 1H), 7.09 (s, 1H), 4.33 - 4.18 (m, 1H), 3.15 - 3.07 (m, 2H), 1.65 - 1.55 (m, 1H), 1.48 -1.42 (m,4H), 1.22 (s, 18H), 0.89- 0.84 (m, 9H); ¹³C NMR (75 MHz, DMSO) δ / ppm 173.6, 160.0, 159.9, 51.8, 41.4, 31.7, 29.4, 29.2, 26.8, 24.8, 23.5, 22.6, 21.9, 14.4; HRMS (MALDI TOF/TOF): calcd for C₂₀H₃₉N₃O₃ [M+H]⁺ 370,3070; found 370,3070.

**C₄-oxalyl-Ile-NH₂ (4-IL):** ¹H NMR (300 MHz, DMSO) δ /ppm 8.85 (t, *J* = 5.9 Hz, 1H), 8.13 (d, *J* = 9.5 Hz, 1H), 7.60 (s, 1H), 7.23 (s, 1H), 4.18 - 4.08 (m, 1H), 3.22 - 3.04 (m, 2H), 1.87 - 1.69 (m, 1H), 1.50 - 1.35 (m, 3H), 1.32 - 1.18 (m, 2H), 1.10 - 0.95 (m, 1H), 0.92 - 0.78 (m, 8H); ¹³C NMR (75 MHz, DMSO) δ / ppm 172.4, 160.0, 159.8, 57.6, 37.4, 31.2, 24.7, 19.9, 15.9, 14.1, 11.4; HRMS (MALDI TOF/TOF): calcd for C₁₂H₂₃N₃O₃ [M+H]⁺ 258,1818; found 258,1820.

**C₆-oxalyl-Ile-NH₂ (6-IL):** ¹H NMR (300 MHz, DMSO) δ /ppm 8.85 (t, *J* = 5.7 Hz, 1H), 8.13 (d, *J* = 9.4 Hz, 1H), 7.63 (s, 1H), 7.22 (s, 1H), 4.20 - 4.06 (m, 1H), 3.20 - 3.05 (m, 1H), 1.88 - 1.70 (m, 1H), 1.50 - 1.35 (m, 3H), 1.24 (s, 6H), 1.08 - 0.96 (m, 1H), 0.91- 0.78 (m, 9H); ¹³C NMR (75 MHz, DMSO) δ / ppm 172.4, 160.0, 159.8, 57.6, 37.4, 31.3, 29.0, 26.4, 24.7, 22.4, 15.9, 14.3, 11.4; HRMS (MALDI TOF/TOF): calcd for C₁₄H₂₇N₃O₃ [M+H]⁺ 286,2131; found 286,2142.

**C₈-oxalyl-Ile*-*NH₂ (8-IL):** ¹H NMR (300 MHz, DMSO) δ /ppm 8.84 (t, *J* = 5.8 Hz, 1H), 8.12 (d, *J* = 9.4 Hz, 1H), 7.60 (s, 1H), 7.22 (s, 1H), 4.22 - 4.05 (m, 1H), 3.19 - 3.04 (m, 2H), 1.86 - 1.66 (m, 1H), 1.52 - 1.36 (m, 3H), 1.24 (s, 10H), 1.10 - 0.95 (m, 1H), 0.91 - 0.77 (m, 9H); ¹³C NMR (75 MHz, DMSO) δ / ppm 172.4, 160.0, 159.8, 57.6, 37.4, 31.7, 29.1, 26.8, 24.7, 22.5, 15.9, 14.4, 11.4; HRMS (MALDI TOF/TOF): calcd for C₁₆H₃₁N₃O₃ [M+H]⁺ 314,2444; found 314,2458.

**C₁₂-oxalyl-Ile-NH₂ (12-IL):** ¹H NMR (300 MHz, DMSO) δ / ppm 8.83 (s, 1H), 8.11 (d, *J=* 9.2 Hz, 1H), 7.63 (s, 1H), 7.21 (s, 1H), 4.21 - 4.04 (m,1H), 3.15 - 3.11 (m, 1H), 1.85 - 1.66 (m, 1H), 1.50 - 1.37 (m, 3H), 1.22 (s, 18H), 1.06 - 0.95 (m, 1H), 0.94 - 0.73 (m, 9H); ¹³C NMR (75 MHz, DMSO) δ /ppm 172.4, 160.0, 159.8, 57.6, 37.4, 31.7, 29.4, 29.1, 26.7, 24.7, 22.5, 15.9, 14.4, 11.4; HRMS (MALDI TOF/TOF): calcd for C₂₀H₃₉N₃O₃ [M+Na]⁺ 392,2889; found 392,2890.

**C₄-oxalyl-Phe-NH₂ (4-P):** ¹H NMR (300 MHz, DMSO) δ /ppm 8.68 (d, *J=* 5.6 Hz, 1H), 8.37 (d, *J* = 8.7 Hz, 1H), 7.59 (s, 1H), 7.28 - 7.16 (m, 6H), 4.55 - 4.40 (m, 1H), 3.16 - 2.96 (m, 4H), 1.50 - 1.32 (m, 2H), 1.30 - 1.15 (m, 2H), 0.85 (t, *J* = 7.2 Hz, 3H); ¹³C NMR (75 MHz, DMSO) δ / ppm 172.4, 159.9, 159.8, 137.9, 129.6, 128.6, 126.9, 54.5, 38.9, 37.7, 31.2, 19.9, 14.0; HRMS (MALDI TOF/TOF): calcd for C₁₅H₂₁N₃O₃ [M+H]⁺ 292,1661; found 292,1658.

**C₆-oxalyl-Phe-NH₂ (6-P):** ¹H NMR (300 MHz, DMSO) δ /ppm 8.67 (t, *J* = 5.9 Hz, 1H), 8.37 (d, *J* = 8.7 Hz, 1H), 7.59 (s, 1H), 7.28 - 7.16 (m, 6H), 4.55 - 4.40 (m, 1H), 3.13 - 2.96 (m, 4H), 1.48 - 1.35 (m, 2H), 1.22 (s, 6H), 0.85 (t, *J=* 6.6 Hz, 3H); ¹³C NMR (75 MHz, DMSO) δ / ppm 172.4, 159.9, 159.8, 138.0, 129.6, 128.5, 126.8, 54.5, 39.3, 37.7, 31.3, 29.1, 26.4, 22.5, 14.4; HRMS (MALDI TOF/TOF): calcd for C₁₇H₂₅N₃O₃ [M+Na]⁺ 342,1794; found 342,1784.

**C₈-oxalyl-Phe-NH₂ (8-P):** ¹H NMR (300 MHz, DMSO) δ / ppm 8.53 (s, 1H), 8.27 (d, *J=* 8.6 Hz, 1H), 7.49 (s, 1H), 7.28 - 7.15 (m, 5H), 7.09 (s, 1H), 4.50 - 4.38 (m, 1H), 3.14 - 2.96 (m, 4H), 1.48 - 1.38 (m, 2H), 1.24 (s, 10H), 0.85 (t, *J=* 6.3 Hz, 3H);¹³C NMR (75 MHz, DMSO) δ / ppm 172.3, 159.9, 159.9, 137.9, 129.6, 128.5, 126.8, 54.4, 37.8, 31.6, 29.0, 26.7, 22.5, 14.3; HRMS (MALDI TOF/TOF): calcd for C₁₉H₂₉N₃O₃ [M+H]⁺ 348,2287; found 348,2274.

**C₁₂-oxalyl-Phe-NH₂ (12-P):** ¹H NMR (300 MHz, DMSO) δ / ppm 8.66 (t, *J* = 5.9 Hz, 1H), 8.36 (d, *J* = 8.7 Hz, 1H), 7.59 (s, 1H), 7.29 - 7.14 (m, 6H), 4.52 - 4.41 (m, 1H), 3.15 - 2.90 (m, 4H), 1.46 - 1.37 (m, 2H), 1.23 (s, 18H), 0.85 (t, *J* = 6.4 Hz, 3H); ¹³C NMR (75 MHz, DMSO) δ / ppm 172.4, 159.9, 159.8, 138.0, 129.6, 128.5, 126.8, 54.5, 39.3, 37.7, 31.8, 29.4, 29.2, 26.7, 22.5, 14.4; HRMS (MALDI TOF/TOF): calcd for C₂₃H₃₇N₃O₃ [M+H]⁺ 404,2913; found 404,2913.

### Determination of Gelling Properties

All gelation experiments were performed in test tubes of 12 mm in diameter.

The tested substance was placed in a test tube, and the oil was added by pipette in 1 mL portions. After each addition the mixture was gently heated until the substance dissolved, and was then allowed to cool spontaneously to room temperature and formation of gel checked by test tube inversion. The procedure is repeated until formation of a loose gel or dissolution is observed. Gelation properties of prepared compounds were tested against various edible oils and the results are collected in Table 1. Gelation efficiency of each gelator toward the specified edible oil is expressed in mL of oil that could be immobilized by 10 mg of the gelator.

All of the prepared vegetable gels are transparent and show thermoreversible gel-to-sol transitions. In experiments sunflower oil (Zvijezda d.o.o.), soybean oil (Fluka), olive oil (Primadonna) were used.

Gelation capabilities in w/o emulsions were monitored at half of the maximal gelling volume (Vₘₐₓ / ml) of sunflower oil that could be immobilized by 10 mg of the specified gelator in addition of 10 wt % of water (Table 2). The recovery time of an opaque w/o emulsion to gelled w/o emulsion state was determined after mechanical agitation (Table 2, recovery time, h or min).

The tested substance was placed in a test tube and the oil was added, the mixture was gently heated until the substance dissolved, and was then allowed to cool spontaneously to room temperature and formation of gel checked by test tube inversion.

After addition of a specified amount of water (10 wt %) to a sunflower gel, a mixture was heated and mixed until solubilization happened, after that w/o emulsion was spontaneously cooled down to room temperature or bellow and formation of gelled w/o emulsion checked by test tube inversion.

All of prepared w/o emulsion gels were opaque and showed thermoreversible gel-to-sol transitions.

**Table 1. Gelation efficiency of prepared compounds toward the specified edible oil expressed as the maximal volume (Vₘₐₓ / ml) of oil that could be immobilized by 10 mg of the gelator.**

| Thixotropic property was determined at half of the maximum gelling volume. | | | | | | |
|---|---|---|---|---|---|---|
| **Compound** | **Sunflower oil V / ml** | Recovery time^{∗}/ h or min | **Soybean oil V / ml** | Recovery time / h or min | **Olive oil V / ml** | Recovery time / h or min |
| **4-V** | **46,6 ml** | 27 min | **43 ml** | 26 min | **26,6ml** | 10 min |
| **5-V** | **35,9ml** | 8 min | **29,2ml** | 6 min | **37,8ml** | 9 min |
| **6-V** | **32,4ml** | 5 min | **29ml** | 1 min | **19,5ml** | **1** min |
| **8-V** | **29,2ml** | 2 h | **36,3ml** | 6 min | **19,4ml** | 6 min |
| **12-V** | **16,4ml** | 16 h | **15,9ml** | 12 min | **18,6ml** | 12 min |
| **4-L** | **25,3ml** | 9 min | **29,7ml** | 6 min | **16,5ml** | 14 min |
| **6-L** | **20,8ml** | 4 min | **17,9ml** | 6 min | **19,3ml** | 18 min |
| **8-L** | **16,3ml** | 6 min | **16,3ml** | 8 min | **19,6ml** | 9 min |
| **12-L** | **15,8ml** | 3 min | **13,2ml** | 3 min | **10ml** | 2 min |
| **4-A** | **51,6ml** | 4 min | **9,7ml** | 3 min | **9,4ml** | 5 min |
| **4-P** | **41,8ml** | 2 min | **41,5ml** | 2 min | **34,9ml** | 4 min |
| **6-P** | **41,5ml** | 9 min | **39,6ml** | 21 min | **39,3ml** | 10 min |
| **8-P** | **39,2ml** | 8 min | **32,5ml** | 22 min | **35,7ml** | 22 min |
| **12-P** | **22,4ml** | 3 h | **12,8ml** | 3 h | **19ml** | 3 h |
| **4-IL** | **38,9ml** | partly soft gel | **33,5ml** | soft gel | **11,6ml** | partly soft gel |
| **6-IL** | **35,5ml** | 7 min | **35,2ml** | 3 min | **43,6ml** | 10 min |
| **8-IL** | **39,6ml** | soft gel | **28,2ml** | viscous solution | **35,9ml** | viscous solution |
| **12-IL** | **26ml** | 50 min | **22,3ml** | 50 min | **33,5ml** | 25 min |

### Determination of thixotropic properties by visual observation

The formed gel or w/o emulsion gel was subjected to external mechanical stress (shaking) till gel is transformed into a liquid sol state. The self-healing process (recovery to gel-state) after standing at room temperature was checked every minute by tube inversion method and visual observation. The time of response necessary to self-heal from sol to gel state was determined at half of the maximum gelling volume per 10 mg of the tested compound (Table 1, column Recovery time (h or min)). The recovery time of a fluidic opaque w/o emulsion to gelled w/o emulsion state was determined after mechanical agitation (Table 2, Recovery time (h or min)). The self-healing process for w/o emulsion (recovery to gel-state) after standing at room temperature was checked every minute by tube inversion method and visual observation.

**Table 2. Gelation capabilities and thixotropic properties of gelled emulsions of sunflower oil with 10% of water expressed at half of the maximal volume (Vₘₐₓ / ml) of oil that could be immobilized by 10 mg of the gelator. The recovery time of the fluidic emulsion to gelled w/o emulsion state was determined after mechanical agitation (h or min).**

| **Compound** | **Gelation of w/o sunflower oil emulsion with 10% of water** | **Recovery time** / h or min |
|---|---|---|
| **4-V** | gel | 16 h |
| **5-V** | gel | no thixotropy high viscosity |
| **6-V** | gel | 5 min |
| **8-V** | gel | 25 min |
| **12-V** | gel | 30 min |
| **4-L** | gel | 6 min |
| **6-L** | gel | 4 min |
| **8-L** | gel | 6 min |
| **12-L** | gel | 3 min |
| **4-A** | gel after 24 h | no thixotropy |
| **4-P** | gel | no thixotropy |
| **6-P** | gel | 15 min |
| **8-P** | gel | 15 min |
| **12-P** | gel | 16 min |
| **4-IL** | soft gel | no thixotropy high viscosity |
| **6-IL** | gel | Soft gel after 18 h |
| **8-IL** | gel | 6 min |
| **12-IL** | gel | 18 h |

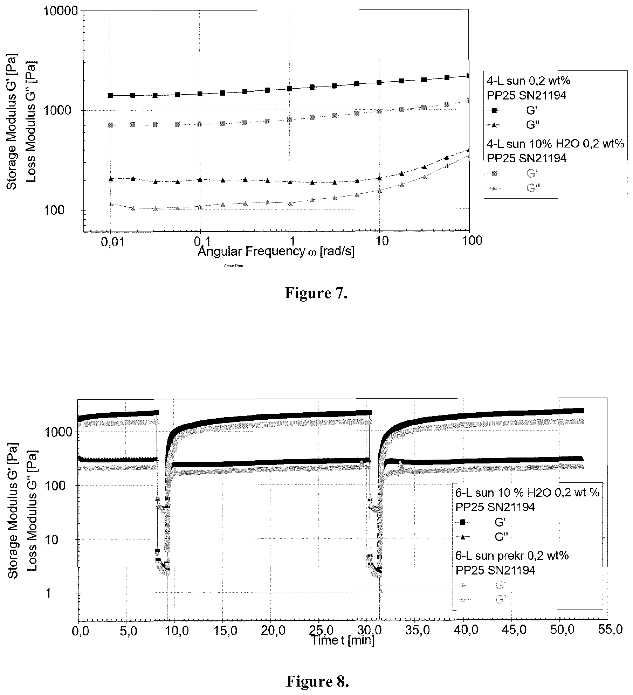

For example, compound **6-L** forms a transparent gel after a heating-cooling process in sunflower oil with a critical gelation concentration (CGC) of 0.05 wt% (which corresponds to 20,8 mL expressed as maximal volume of oil that could be immobilized by 10 mg of compound **6-L**, see Table 1). When treated with external mechanical stress, the **6-L** sunflower oil gel lost most of its viscosity and transformed into a sol; after resting for 4 min at room temperature, the gel completely regenerated. The self-healing process after the gel to sol transition, which was brought about by mechanical stress, can be repeated many times (Figure 8., Thixotropic rheological measurements). Stability and transparency of all examined gels of vegetable oils were monitored through period of one year. All tested gels in different vegetable oils were stable even after a period of one year. In addition, for example, the **6-L** sunflower oil gel is capable of forming gelled w/o emulsion as shown in Table 2. When treated with external mechanical stress, a hand shaking, the gelled **6-L** sunflower oil emulsion (10 % of water) lost most of its viscosity and transformed into a fluid emulsion state; after resting for 4 min at room temperature, the gelled w/o emulsion completely regenerated. The self-healing process of emulsion to gelled emulsion state can be repeated many times (Figure 8. Thixotropy rheological measurements).

Gelation efficiencies of the tested compounds toward the specified edible oil expressed as the minimal concentration of the gelators were in the range of 0,1 wt% till 0,02 wt%. Even at minimal concentrations of the gelators in different oils very prominent self-healing behaviour was noticed, especially for 4-P, 12-L, 6-L, 8-L, 4-A and 6-V compounds who showed thixotropic behaviour even after few minutes standing at room temperature. Gelation of water-in-oil emulsions was even greater challenge, because usually compounds who are capable to gel lipophilic solvents, such as oil, tend to precipitate in a water surrounding. According to present invention gelled w/o emulsions with 12-L, 6-L, 6-V, 4-L, 8-L, 8-IL showed thixotropic (self-healing) properties at room temperature only after few minutes (Table 2).

### Rheological investigation

The elastic (G') and loss (G") moduli of the 0.2 wt% organogels were determined with a mechanical spectrometer (Anton Paar MCR 302, Stuttgart, Germany), using a steel plate-plate geometry (PP25, Anton Paar, Graz-Austria) equipped with a true-gap system and the data were collected using RheoCompass software. The sample temperature was controlled through a Peltier temperature control located on the base of the geometry and with a Peltier-controlled hood (H-PTD 200). An aliquot of a 0.2 wt% gel sample was placed on the base plate of the rheometer, and the plate was set using the true-gap function of the software. Thus, after 5 min at 15 °C, the G' and G" moduli were measured always within the linear viscoelastic region (LVR). After 5 min at 15 °C, the yield stress of the organogels was determined by applying a strain (γ) sweep between 0,01 % and 100%. Shear strain or shear deformation (γ) is defined in two-plates model as deflection path (s) divided by shear gap width (h). The unit of shear deformation is dimensionless, usually stated as a percentage. Rheological properties of a gel material are independent of strain up to yield strain, and beyond yield strain the rheological behaviour is nonlinear. Frequency sweeps (0.01-100 Hz) were subsequently performed at 15 °C at a strain value within LVR to investigate the time-dependent deformation behaviour of gels.

The mechanical properties of gels composed of vegetable oils and gelators are described using oscillatory rheology.

### Amplitude sweep

The storage modulus G' (Pa) represents the elastic portion of the viscoelastic behaviour, which describes the solid-state behaviour of the sample. The loss modulus G" (Pa) characterizes the viscous portion of the viscoelastic behaviour, which can be seen as the liquid-state behaviour of the sample. Storage modulus G' represents the stored deformation energy and loss modulus G" characterizes the deformation energy lost (dissipated) through internal friction when flowing. Viscoelastic solids with G' > G" have a higher storage modulus than loss modulus.

Typically, for gels, the elastic component dominates (G') over viscous component (G") at small applied shear and attains a plateau in the linear response region (LVR or linear viscoelastic region). The end of LVR is marked by the first point where the G' varies by 10 % of the LVR value and the corresponding stress at this point is referred to as the critical stress. As the applied shear increases further, a permanent deformation (yielding) of the materials may occur and the corresponding stress value at this point is referred to as yield stress. The yield stress represents the onset of nonlinearity (and hence structure breakdown), while flow stress represents the transition from solid- to liquid-like behaviour (G' = G") and the zone spanning of these two events is referred to as the yield zone.

The yield point is the lowest shear stress, above which a sample shows an irreversible structural change; below the yield point it shows reversible elastic or viscoelastic behaviour.

The flow point is the critical shear stress value above which a sample rheologically behaves like a liquid; below the flow point it shows elastic or viscoelastic behaviour.

### Results

Strain sweep tests for gelators of the present invention were performed from γ = 0.01 to 100% at ω = 10 rad s⁻¹ with a very low concentration (0.2 wt %) of compound of the invention at 15 °C. The G' and G" values remained approximately independent of applied strain up to 0.1 %.

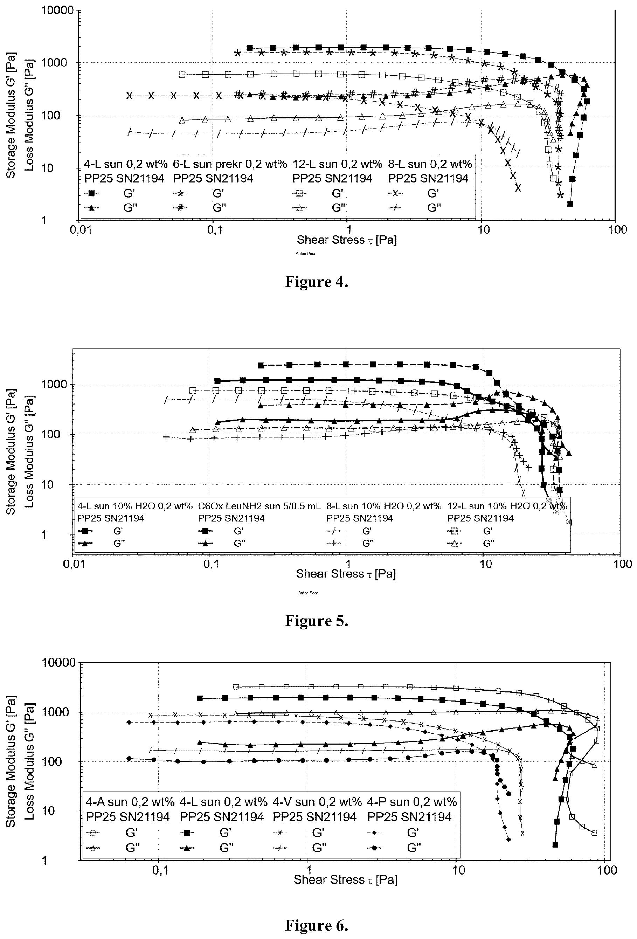

The storage modulus (G') values (determined at LVR, 10 rad/s and 15 °C; Figure 4) of sunflower oil gels containing 0.2 wt% of the compound 4-L or 6-L were 1900 Pa and 1500 Pa, respectively (Figure 4.). The storage modulus (G') values of sunflower oil gels containing 0.2 wt% of the compound 12-L or 8-L were 600 and 232 Pa, respectively.

The yield stress values observed for the sunflower oil gels containing 0,2 wt % of the compound 4-L, 6-L, 12-L or 8-L were 25,3 Pa; 14,7 Pa; 9,5 Pa and 3,7 Pa, respectively. The flow point values for the sunflower oil gels containing 0,2 wt % of the compound 4-L, 12-L, 6-L or 8-L were 40,7 Pa; 24 Pa; 22,2 Pa and 10,4 Pa respectively.

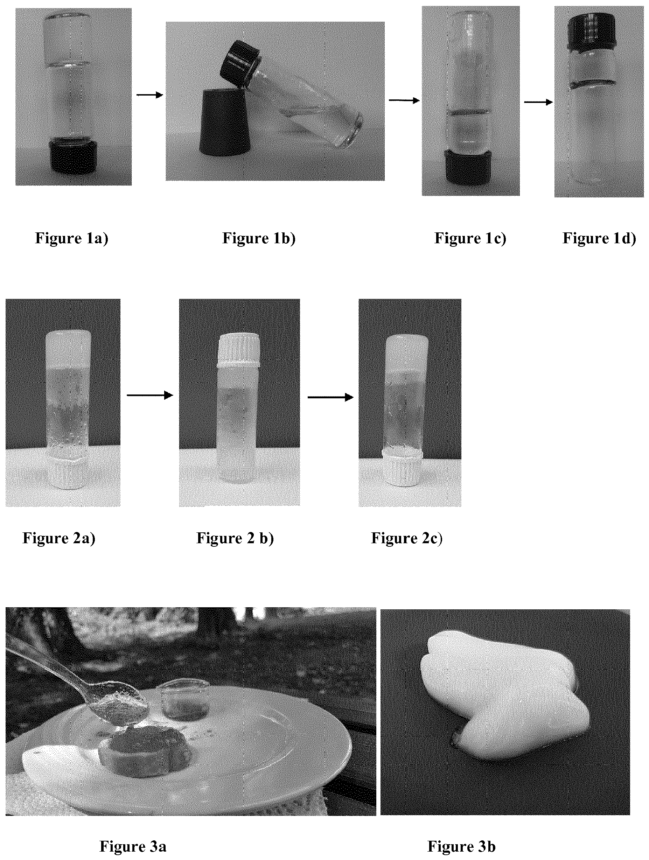

The rheologically determined quantity loss factor (tan(δ) = G"/G') determines the relative elasticity of viscoelastic materials. The gels formed in all investigated oils showed relatively low tan(δ) values in range from 0,12 till 0,19 which is indicative of a high relative elastic modulus for the gels in all examined vegetable oils. The gels with a value of tan(δ) = 0,1 belong to stiff gels. The most of investigated gels in sunflower oil, soybean oil and olive oil were moderately stiff gels even at the low concentration of 0.2 wt%. Storage modulus (G') values and flow point values for higher concentrations of gelators were considerably larger, for example, sunflower oil gel containing 0,5 wt% of the compound 6-L exhibits G' = 10000 Pa and flow point τ = 90 Pa, respectively. Amplitude sweeps of gelled water in sunflower oil emulsions (water content from 10% to 50%) for gels with 0,2 wt% of the compounds 4-L, 6-L, 12-L or 8-L showed similar or even more prominent viscoelastic behaviour compared with pure vegetable oil gels (Figure 5). Flow point values for investigated gels (Table 1. and 2.) at 0,2 wt% of gelators were in the range from 11 Pa to 33 Pa and the storage modulus (G') values (determined at LVR, 10 rad/s and 15 °C; Figure 2) were from 477 Pa to 2340 Pa.

Gels of oils and gelled emulsions subjected to rheological measurements were moderate to strong gels examined at low concentrations from 0.5 till 0.05 wt%.

Rheological properties of gels with compound of the invention having the same aliphatic chain (butyl) but different aminoacid side chains are shown in Figure 6. Storage modulus (G') value (determined at LVR, 10 rad/s and 15 °C; Figure 6) of sunflower oil gel containing 0.2 wt% of the compound 4-A, 4-L, 4-V or 4-P were 3200, 1900, 867 and 624 Pa, respectively. The largest yield stress value was observed for the sunflower oil gel containing 0,2 wt % of compound 4-A (36,4 Pa). The flow point values of sunflower oil gels with 0,2 wt % of compounds 4-A, 4-L, 4-V or 4-P were 65 Pa; 40,7 Pa; 23,5 Pa and 16,2 Pa, respectively.

### Frequency sweep

Frequency sweep studies (ω = 0.01-100 rad/s at 0.1% strain) of the gels at 15 °C indicate that the storage modulus (G') and loss modulus (G") values are mostly independent of the applied frequency within the linear viscoelastic regions (LVRs) (Figure 7, an example of the 4-L sunflower oil gel and gelled water in oil emulsion). All investigated compounds aforementioned in the Tables 1. and 2. in form of gelled oils and water in oil emulsions according to rheological measurements are true gels.

Frequency sweep for gel containing 0,2 wt% of the compound 4-L showed less relative elasticity in gelled w/o emulsion at higher frequencies (100 rad/s, loss factor 0,28) compared with loss factor determined for pure sunflower oil gel (100 rad/s, loss factor 0,18). The loss factor at the frequency of 0,01 rad/s for both the 4-L sunflower oil gel and the gelled w/o emulsion amounts 0,15. This particular behaviour observed in the frequency sweep was characteristic for the most of investigated gelators mentioned in the Table 1. and Table 2.

Frequency sweeps at very low frequency values (0,01-0,001 rad/s) were performed to evaluate a material at rest, during which different oil gels and w/o gelled emulsions showed exceptional stability.

### Thixotropic properties of sunflower oil gels and gelled w/o emulsions

The possibility that the gelators of different vegetable oils and gelled w/o emulsions mentioned in Tables 1. and 2. are thixotropic was explored.

In the thixotropic experiments, rheological measurements were conducted on fresh gels at 15 °C under initial conditions at which they were in their linear viscoelastic regimes (a strain of 0.1 % and angular frequency of 10 rad/s) for 500 s to establish baseline values for G' and G". In studies with organogels we have observed viscoelastic recovery after the cessation of destructive strain (Figure 8).

For the first 500 s, strain = 0.1 % and angular frequency = 10 rad/s; for the next 60 s, strain = 100 % and angular frequency = 10 rad/s; thereafter, strain 0.1 % and angular frequency = 10 rad/s. Figure 8 shows the evolution of G' and G" for the sunflower oil gel containing 0.2 wt % of compound 6-L and gelled w/o emulsion (10 % of water) after applying a 100 % strain, a condition that leads to loss of its viscoelasticity. After that, the original conditions were reapplied in order to monitor the recovery of the viscoelastic properties of the gels.

Approximately, 35 % of the original G' values were recovered in less than 30 s. Then, after 5 minutes the recovery of viscoelasticity were 77 % for the sunflower oil gel and 69 % for gelled w/o emulsion (10 % of water). The recovery of 90 % of the original G' value in sunflower oil gel and gelled w/o emulsion were obtained after 11 minutes and 15 minutes, respectively. Nevertheless, for most of the investigated samples with thixotropic properties (Table 1. and Table 2.) 50 % of the original G' values were recovered in less than 2 minutes.

The recovery of G' and G" values in 2 consecutive thixotropic recovery cycles was investigated. After application, again of the 100 % destructive strain, usually about 90 % of the previous G' value was recovered.

## Claims

1. The composition with gelling properties comprising a compound of Formula (I) 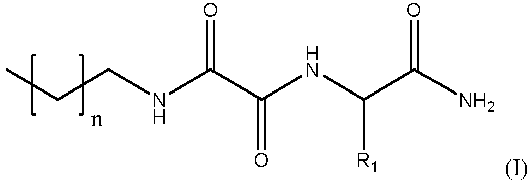 and a vegetable oil;
wherein R¹ is selected from -CH₃, -CH(CH₃)₂, -CH₂CH(CH₃)₂, -CH(CH₃)CH₂CH₃, -CH₂Ph and -Ph;
n is an integer from 0-14.

2. The composition according to claim 1 comprising a compound of Formula (Ia) 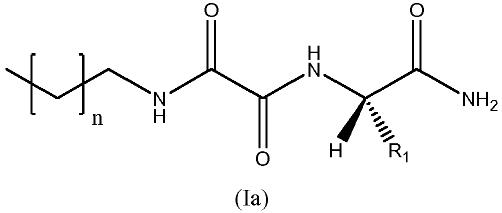 and a vegetable oil;
wherein R¹ is selected from -CH₃, -CH(CH₃)₂, -CH₂CH(CH₃)₂, -CH(CH₃)CH₂CH₃, -CH₂Ph and -Ph;
n is an integer from 0-14.

3. A composition according to claim 1 or 2 wherein the composition is in the form of gel.

4. A composition according to any of claims 1 to 3 wherein the oil is edible oil selected from palm oil, sunflower oil, olive oil, soybean oil, linseed oil, rapeseed oil, corn oil, pumpkin seed oil, sesame oil, safflower oil, castor, peanut oil or combination thereof.

5. A composition according to any of claims 1 to 3 wherein the oil is base cosmetic oil selected from sweet almond oil, linseed oil, grape seed oil, avocado oil, apricot oil, olive oil, sesame oil, rapeseed oil, sunflower oil, jojoba oil, castor oil, borage seed oil, argan oil, avocado oil, calendula oil, evening primrose oil, hazelnut oil, walnut oil, peanut oil, macadamia oil, coconut oil, rose hip seed oil, wheat germ oil, St. John's wort oil, blueberry seed oil, black cumin seed, rice bran oil or combination thereof.

6. A composition according to any of claims 1 to 3 wherein the oil is selected from sunflower oil, olive oil, soybean oil or combination thereof.

7. A composition according to any of claims 1 to 6 wherein n is an integer from 2 to 10.

8. A composition according to any of claims 1 to 7 wherein R¹ is selected from -CH₃, -CH(CH₃)₂, -CH₂CH(CH₃)₂, -CH(CH₃)CH₂CH₃ and -CH₂Ph.

9. A composition according to any of claims 1 to 8 further comprising water.

10. A composition according to claim 9 wherein composition is gelled water in oil emulsion.

11. A composition according to claim 9 or 10 wherein water is present in the composition in the concentration to about 10 wt % relative to the total weight of the composition.

12. A composition according to claim 9 or 10 wherein water is present in the composition in the concentration to about 50 wt % relative to the total weight of the composition.

13. A composition according to any of claims 1 to 12 wherein composition exhibits a thixotropic property.

14. A composition according to any of claims 1 to 13 further comprising either:
(a) a food;
(b) a cosmetically acceptable ingredient or
(c) a pharmaceutically acceptable ingredient.

## Patentansprüche

1. Zusammensetzung mit Geliereigenschaften, die eine Verbindung der Formel (I) 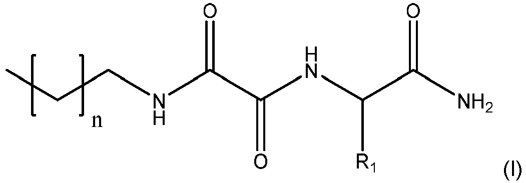 und ein Pflanzenöl enthält;
wobei R¹ aus -CH₃, -CH(CH₃)₂, -CH₂CH(CH₃)₂, -CH(CH₃)CH₂CH₃, -CH₂Ph und -Ph ausgewählt ist;
n ist eine ganze Zahl von 0-14.

2. Zusammensetzung nach Anspruch 1, die eine Verbindung der Formel (la) 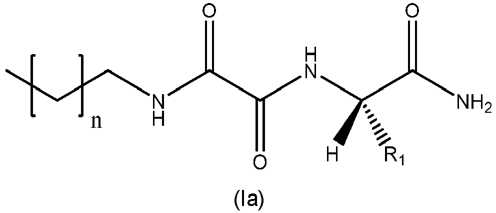 und ein Pflanzenöl enthält;
wobei R¹ aus -CH₃, -CH(CH₃)₂, -CH₂CH(CH₃)₂, -CH(CH₃)CH₂CH₃, -CH₂Ph und -Ph ausgewählt ist;
n ist eine ganze Zahl von 0-14.

3. Zusammensetzung nach Ansprüchen 1 oder 2, wobei die Zusammensetzung in Form eines Gels vorliegt.

4. Zusammensetzung nach einem der Ansprüche 1 bis 3, wobei das Öl ein Speiseöl ist, ausgewählt aus Palmöl, Sonnenblumenöl, Olivenöl, Sojaöl, Leinöl, Rapsöl, Maisöl, Kürbiskernöl, Sesamöl, Distelöl, Rizinusöl, Erdnussöl oder einer Kombination davon.

5. Zusammensetzung nach einem der Ansprüche 1 bis 3, wobei das Öl ein kosmetisches Basisöl ist, ausgewählt aus Süßmandelöl, Leinöl, Traubenkernöl, Avocadoöl, Aprikosenöl, Olivenöl, Sesamöl, Rapsöl, Sonnenblumenöl, Jojobaöl, Rizinusöl, Borretschsamenöl, Arganöl, Avocadoöl, Calendulaöl, Nachtkerzenöl, Haselnussöl, Walnussöl, Erdnussöl, Macadamiaöl, Kokosnussöl, Hagebuttensamenöl, Weizenkeimöl, Johanniskrautöl, Heidelbeersamenöl, Schwarzkümmelöl, Reiskleieöl oder einer Kombination davon.

6. Zusammensetzung nach einem der Ansprüche 1 bis 3, wobei das Öl aus Sonnenblumenöl, Olivenöl, Sojaöl oder einer Kombination davon ausgewählt ist.

7. Zusammensetzung nach einem der Ansprüche 1 bis 6, wobei n eine ganze Zahl von 2 bis 10 ist.

8. Zusammensetzung nach einem der Ansprüche 1 bis 7, wobei R¹ aus -CH₃, -CH(CH₃)₂, -CH₂CH(CH₃)₂, -CH(CH₃)CH₂CH₃ und CH₂-Ph ausgewählt ist.

9. Zusammensetzung nach einem der Ansprüche 1 bis 8, die weiterhin Wasser enthält.

10. Zusammensetzung nach Anspruch 9, wobei die Zusammensetzung eine gelierte Wasser-in-ÖI-Emulsion ist.

11. Zusammensetzung nach Ansprüchen 9 oder 10, wobei Wasser in der Zusammensetzung in einer Konzentration von etwa 10 Gew.-%, in Bezug auf das Gesamtgewicht der Zusammensetzung, vorhanden ist.

12. Zusammensetzung nach Ansprüchen 9 oder 10, wobei Wasser in der Zusammensetzung in einer Konzentration von etwa 50 Gew.-%, in Bezug auf das Gesamtgewicht der Zusammensetzung, vorhanden ist.

13. Zusammensetzung nach einem der Ansprüche 1 bis 12, wobei die Zusammensetzung eine thixotrope Eigenschaft aufweist.

14. Zusammensetzung nach einem der Ansprüche 1 bis 13, die außerdem entweder:
(a) ein Lebensmittel;
(b) einen kosmetisch akzeptablen Inhaltsstoff oder
(c) einen pharmazeutisch akzeptablen Inhaltsstoff
enthält.

## Revendications

1. Une composition ayant des propriétés gélifiantes comprenant un composé de formule (I) 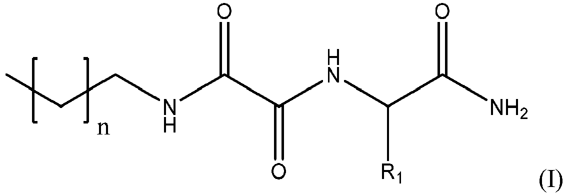 et une huile végétale ;
dans laquelle R¹ est choisi parmi -CH₃, -CH(CH₃)₂, -CH₂CH(CH₃)₂, -CH(CH₃)CH₂CH₃, - CH₂Ph et-Ph ;
n est un nombre entier de 0 à 14.

2. Une composition selon la revendication 1 comprenant un composé de formule (la) 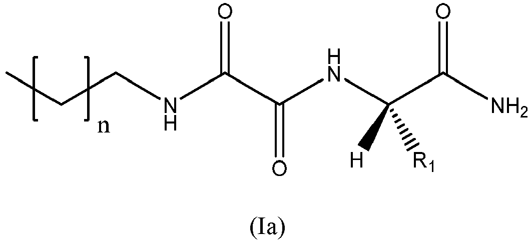 et une huile végétale ;
dans laquelle R¹ est choisi parmi -CH₃, -CH(CH₃)₂, -CH₂CH(CH₃)₂, -CH(CH₃)CH₂CH₃, - CH₂Ph et-Ph ;
n est un nombre entier de 0 à 14.

3. Une composition selon la revendication 1 ou 2, dans laquelle la composition est sous forme de gel.

4. Une composition selon l'une des revendications 1 à 3 dans laquelle l'huile est une huile comestible choisie parmi l'huile de palme, l'huile de tournesol, l'huile d'olive, l'huile de soja, l'huile de lin, l'huile de colza, l'huile de maïs, l'huile de pépins de courge, l'huile de sésame, l'huile de carthame, l'huile de ricin, l'huile d'arachide ou une combinaison de celles-ci.

5. Une composition selon l'une des revendications 1 à 3, dans laquelle l'huile est une huile cosmétique de base choisie parmi l'huile d'amande douce, l'huile de lin, l'huile de pépins de raisin, l'huile d'avocat, l'huile d'abricot, l'huile d'olive, l'huile de sésame, l'huile de colza, l'huile de jojoba, l'huile de ricin, l'huile de graines de bourrache, l'huile d'argan, l'huile d'avocat, l'huile de calendula, l'huile d'onagre, l'huile de noisette, l'huile de noix, l'huile d'arachide, l'huile de macadamia, l'huile de noix de coco, l'huile de graines de rose musquée, l'huile de germe de blé, huile de millepertuis, huile de graines de myrtille, huile de nigelle, huile de son de riz ou une combinaison de celles-ci.

6. Une composition selon l'une des revendications 1 à 3, dans laquelle l'huile est choisie parmi l'huile de tournesol, l'huile d'olive, l'huile de soja ou une combinaison de celles-ci.

7. Une composition selon l'une des revendications 1 à 6, dans laquelle n est un nombre entier de 2 à 10.

8. Une composition selon l'une des revendications 1 à 7 dans laquelle R¹ est choisi parmi - CH₃, -CH(CH₃)₂, -CH₂CH(CH₃)₂, -CH(CH₃)CH₂CH₃ et -CH₂Ph.

9. Une composition selon l'une des revendications 1 à 8 comprenant en outre de l'eau.

10. Une composition selon la revendication 9, dans laquelle la composition est une émulsion gélifiée d'eau dans l'huile.

11. Une composition selon la revendication 9 ou 10, dans laquelle l'eau est présente dans la composition à la concentration d'environ 10 % en poids par rapport au poids total de la composition.

12. Une composition selon la revendication 9 ou 10, dans laquelle l'eau est présente dans la composition à la concentration d'environ 50 % en poids par rapport au poids total de la composition.

13. Une composition selon l'une des revendications 1 à 12, dans laquelle la composition présente une propriété thixotropique.

14. Une composition selon l'une des revendications 1 à 13 comprenant en outre soit :
(a) un aliment ;
(b) un ingrédient cosmétiquement acceptable ou
(c) un ingrédient pharmaceutiquement acceptable.
